# EUROPÄISCHE PATENTANMELDUNG

(11) **EP 3 662 854 A1**
(43) Veröffentlichungstag der Anmeldung: **10.06.2020**
(21) Anmeldenummer: 18210346.5
(22) Anmeldetag: 05.12.2018
(51) Int. Cl.: A61B 18/14, A61B 18/04, A61B 18/12, A61B 18/16

(54) **PLASMABEHANDLUNGSEINRICHTUNG**

(71) Anmelder: Erbe Elektromedizin GmbH, 72072 Tübingen (DE)
(72) Erfinder: Zenker, Matthias, 72074 Tübingen (DE)
(74) Vertreter: Rüger Abel Patentanwälte PartGmbB

(57) **Zusammenfassung**

Der Plasmasensor überwacht während der Behandlungsphase den Zustand des Plasmas charakterisierende Größen und/oder deren Veränderung, um so eine sich ankündigende oder bereits erfolgte Unterbrechung des Plasmas zu erkennen und diese im Idealfall bereits im Vorfeld durch Änderung der Spannungsform zu verhindern. Die genannten Mechanismen können von der Steuereinrichtung (22) auch während eines Pulspakets genutzt werden. Die Länge jedes Pulspakets wird bei jedem Wechsel der Spannungsform gemäß deren Eigenschaften angepasst, um eine gleichbleibende mittlere Leistung sicherzustellen.

## Beschreibung

Die Erfindung betrifft eine Plasma-Behandlungseinrichtung mit verbesserter Handhabung, insbesondere eine solche Argon-Plasma-Behandlungseinrichtung. Die Plasmabehandlung kann eine Koagulation, ein Abtöten, eine Verdampfung oder ein Trennen von Gewebe sein. Ebenfalls möglich ist eine Kaltplasmabehandlung, beispielsweise mit dem Ziel einer Desinfektion oder einer Wundbehandlung.

Aus der DE 696 320 80 T2 ist ein endoskopischer Argon-Plasma-Endoskopiekoagulator bekannt, der einen flexiblen Schlauch aufweist, in dessen Lumen eine Elektrode angeordnet ist. Die Elektrode ist über eine Leitung proximal an einem HF-Generator angeschlossen, der die Elektrode mit einer Hochfrequenzspannung beaufschlagt. Die flexible Sonde ist in ein Lumen eines Endoskops eingesetzt, das an seinem distalen Ende eine zu einer Beobachtungsoptik gehörende Linse aufweist. Das Sichtfeld der Linse ist so ausgerichtet, dass der Behandlungsort und somit der von der Elektrode ausgehende Funke bzw. Plasmastrahl im Sichtfeld des Behandlers liegt.

In ähnlicher Weise ist die Koagulationsvorrichtung nach der WO 98/25530 ausgebildet.

Die EP 2 231 046 B1 befasst sich mit der Spannungsversorgung eines solchen Plasmaapplikators, der in den Arbeitskanal eines flexiblen Endoskops einzusetzen ist. Als kritischer Moment wird dort die kurze Zeitspanne nach dem Zünden eines Funkens betrachtet, während der ein hoher, in dieser Höhe unerwünschter elektrischer Strom fließen kann. Zur Abhilfe wird der Elektrode ein Widerstandselement vorgeschaltet.

Aus der DE 50 105 427 A1 ist ein Generator bekannt, bei dem die abgegebene Leistung durch Veränderung der Spannungsform, insbesondere der Modulation bzw. des Puls-Pausen-Verhältnisses, eingestellt wird. Die Spitzenspannung und damit auch die Intensität eines Lichtbogens bzw. Funkens werden konstant gehalten.

Aus der EP 1 307 154 B1 ist ein Generator mit einstellbarer Begrenzung der Ausgangswirkleistung bekannt, bei dem zur Leistungsmodulation das Puls/Pausen-Verhältnis der modulierten Hochfrequenzspannung variiert wird.

Grundsätzlich wünscht der Behandler der Plasmabehandlung bei der Aktivierung eine sichere und sofortige Ausbildung eines Funkens bzw. eines Plasmas. Jedoch sollen unerwünschte Auswirkungen der Plasmazündung und/oder des durch das Plasma übertragenen elektrischen Stroms vermieden oder minimiert werden. Solche unerwünschten Auswirkungen können beispielsweise eine Blendung des Behandlers, eine zu starke Geräuschentwicklung, eine zu starke, zu schnelle oder zu großflächige thermische Schädigung des Gewebes, eine Punktierung desselben, eine zu starke elektromagnetische Störung oder eine Kombination daraus sein.

Davon leitet sich die Aufgabe ab, eine Plasmabehandlungseinrichtung mit verbesserter Handhabung zu schaffen.

Diese Aufgabe wird mit der Einrichtung zur Plasmabehandlung nach Anspruch 1 und/oder einem Verfahren nach Anspruch 15 gelöst:

Zu der erfindungsgemäßen Einrichtung gehört ein Generator, der zur Erzeugung einer hochfrequenten Wechselspannung (Hochfrequenzspannung) in verschiedenen Einstellungen eingerichtet ist. In diesen verschiedenen Einstellungen hat die Hochfrequenzspannung verschiedene Spannungseigenschaften und/oder der Generator hat unterschiedliche elektrische Eigenschaften. Die unterschiedlichen elektrischen Eigenschaften des Generators bzw. seine Einstellungen können z.B. dessen Innenwiderstand, die Spitzenspannung, die Modulationsform der Hochfrequenzspannung, die Modulationsfrequenz der Hochfrequenzspannung, den Maximalstrom der Hochfrequenzspannung oder dergleichen betreffen. Die unterschiedlichen Einstellungen des Generators bzw. die unterschiedlichen Spannungseigenschaften führen zu unterschiedlichen Zündfähigkeiten und/oder unterschiedliche Plasmaerhaltungsfähigkeiten.

Der Generator kann zu Beginn eines Pulses in einer Zündbetriebsart und danach, während des Pulses, in einer auf den gewünschten Effekt abgestimmten Betriebsart betrieben werden. Z.B. kann der Generator zu Beginn eines Pulses in unmoduliertem Betrieb (CW) und danach, während des Pulses, in einer anderen, z.B. modulierten (gepulsten) Betriebsart betrieben werden. Diese Betriebsart kann nach dem gewünschten Effekt ausgerichtet sein. Z.B. kann es zur Mukosaablation gewünscht sein, eine großflächige flache Koagulation zu erzielen.

Es ist aber auch möglich, die Betriebsart zu Beginn eines Pulses beim Zünden und die nachfolgende Betriebsart beim Erhalt des Plasmas zur Bewirkung eines Effekts nach anderen Kriterien festzulegen. Z.B. können in beiden Phasen die Zündfähigkeit und die Plasmaerhaltung nach verschiedenen Kriterien, z.B. minimalem Nebeneffekt oder anderen Kriterien eingerichtet werden.

Unter der Zündfähigkeit wird dabei jedes Maß verstanden, das anzeigt, ob bei den gegeben Verhältnissen überhaupt eine Plasmazündung stattfindet und, wenn ja, wie weit der Elektrodenabstand (bzw. Elektroden-Gewebe-Abstand) vergrößert werden kann, bis keine Zündung mehr stattfindet. Unter den gegebenen Verhältnissen werden alle den Zündvorgang beeinflussenden physikalischen Größen verstanden, wie beispielsweise Elektrodengröße und Elektrodenform, Elektrodenabstand, Gaszusammensetzung, Feuchtigkeit des Gewebes, Art des Gewebes, Temperatur des Gewebe, des Gases, der Elektrode(n) usw.

Unter der Plasmaerhaltungsfähigkeit wird jedes Maß verstanden, das anzeigt, ob sich bei den gegeben Verhältnissen ein Plasma aufrechterhalten lässt und, wenn ja, wie weit der Elektrodenabstand (bzw. Elektroden-Gewebe-Abstand) vergrößert werden kann, bis das Plasma verlischt.

Hinsichtlich der Plasmaerhaltung werden unter den gegebenen Verhältnissen alle die Plasmaerhaltung beeinflussenden physikalischen Größen verstanden, wie beispielsweise Elektrodengröße und Elektrodenform, Elektrodenabstand, Gaszusammensetzung, Feuchtigkeit des Gewebes, Art des Gewebes, Temperatur des Gewebe, des Gases, der Elektrode(n) usw.

An den Generator ist ein mit einem Behandlungsstrom zu versorgendes Instrument oder eine Sonde mit mindestens einer Elektrode angeschlossenen oder anschließbar. Über die Elektrode wird ein Plasma gespeist, das zur Einwirkung auf ein biologisches Gewebe dient oder geeignet ist.

An den Generator und/oder das Instrument ist ein Plasmasensor angeschlossen, mittels dessen das Verhalten des Plasmas feststellbar ist. An den Plasmasensor und an den Generator ist eine Steuereinrichtung zur Beeinflussung der Einstellung des Generators angeschlossen. Die Beeinflussung der Einstellung erfolgt dabei anhand der von der Steuereinrichtung ermittelten Zündfähigkeit und/oder Plasmaerhaltungsfähigkeit. Die Steuereinrichtung ist dabei derart ausgebildet, dass sie anhand des durch den Plasmasensor erfassten Verhaltens des Plasmas eine geeignete Einstellung für den Generator festlegt. Die Steuereinrichtung wählt dabei, wenn sie ein sicheres Zünden und/oder einen sicheren Plasmaerhalt festgestellt hat, testweise eine Einstellung mit niedrigerer Zündfähigkeit und/oder niedrigerer Plasmaerhaltung. Wird dabei unter den gegebenen Einsatzbedingungen des angeschlossenen Instruments weiterhin sicher ein Funke gezündet und/oder ein Plasma erhalten, wechselt die Steuereinrichtung nicht zu der vorherigen Einstellung des Generators zurück. Weiter kann vorzugsweise vorgesehen sein, dass der Generator zu einer Einstellung mit verbesserter Zündfähigkeit oder Plasmaerhaltungsfähigkeit, z.B. zu der vorherigen Einstellung des Generators zurück wechselt, wenn ein Plasma bei der testweise vorgenommenen Einstellung nicht mehr sicher gezündet worden ist und/oder das Plasma nicht sicher aufrechterhalten wird.

Der Generator ist in seinen unterschiedlichen Einstellungen z.B. zur Erzeugung von Hochfrequenzspannung unterschiedlichen Spannungsformen eingerichtet. Der Generator kann dabei so eingerichtet sein, dass er zwischen zwei oder mehreren Spannungsformen umschalten oder auch die Spannungsform stufenlos variieren kann. Unter einer Spannungsform kann jedes Merkmal der Hochfrequenzspannung verstanden werden, das die Zündfähigkeit und/oder die Plasmaerhaltungsfähigkeit derselben beeinflusst. Insbesondere kann die Spannungsform wenigstens eine der nachgenannten Größen betreffen: Die Frequenz, die Spitzenspannung, den Mittelwert, den Effektivwert, die Kurvenform, die Hüllkurve, mit der die Hochfrequenzspannung moduliert ist, die Amplitude der Hüllkurve, die Form und/oder Frequenz derselben, das Puls/Pause-Verhältnis (bei Ein/Aus-getasteter Hochfrequenzspannung) oder dergleichen Größen. Die Hochfrequenzspannung kann ein- oder mehrfach gepulst sein, wobei sich die verschiedenen Spannungsformen durch die Länge der Impulse und/oder die Länge der zwischen Impulsen liegenden Pausen unterscheiden können. Unter den verschiedenen Spannungsformen kann sich auch eine Spannungsform finden, in der der Generator ungepulst, d.h. mit Pausenzeit Null arbeitet. Die gepulsten Spannungsformen umfassen typischerweise einen Puls mit einer oder mehreren hochfrequenten Schwingungen, die am Ende des Pulses gedämpft abklingen. Diese und weitere Möglichkeiten, durch die Spannungsform die Zündfähigkeit und/oder die Plasmaerhaltungsfähigkeit zu beeinflussen, können auch kombiniert werden.

Prinzipiell können sich die verschiedenen von dem Generator in verschiedenen Einstellungen bereitgestellten Spannungsformen durch unterschiedliche Merkmale unterscheiden, beispielsweise durch die Spitzenspannung, den Mittelwert, den Effektivwert, den Gleichanteil, die Wellenform, die Hüllkurve, die Amplitude der Hüllkurve, die Form derselben, den zeitlichen Verlauf, die Frequenz oder andere elektrische und/oder zeitliche Größen, bei Ein/Ausgetasteter Hochfrequenzspannung das Puls/Pause-Verhältnis und/oder Kombinationen daraus.

Bei einer bevorzugten Ausführungsform ist die Hochfrequenzspannung gepulst und die verschiedenen Spannungsformen unterscheiden sich vorwiegend oder ausschließlich durch Dauer der Pulse und/oder Dauer der zwischen Pulsen liegenden Pausen. Dabei kann die Pulsfrequenz konstant oder variabel sein. Ebenso kann das Puls/Pause-Verhältnis (Tastverhältnis) konstant oder variabel sein und zum Beispiel auch das Puls/Pause-Verhältnis von 100:0 ("Dauerstrich") umfassen.

Die verschiedenen Einstellungen des Generators führen typischerweise zu Behandlungsströmen, mit denen sich gleiche oder unterschiedliche gewünschte physiologische Wirkungen an dem Gewebe erzielen lassen. Zugleich führen sie aber auch zu unterschiedlichen Nebeneffekten, wie z.B. Lichterscheinungen, Schallerscheinungen, Druckimpulsen etc. Auch kann mittels der Behandlungsströme und der unterschiedlichen Generatoreinstellungen gezielt die Plasmatemperatur beeinflusst werden, bis hin zur Erzeugung eines Kaltplasmas, das keinen thermischen Gewebeeffekt zeigt.

Das zu der Einrichtung gehörige Instrument weist mindestens eine Elektrode auf, die über eine Leitung mit dem Generator verbunden oder verbindbar ist. Das zur Behandlung vorgesehene elektrisch leitende Plasma überbrückt bei Aktivierung des Instruments, z.B. in Form eines Funkens, den Abstand zwischen Elektroden oder zwischen der Elektrode und einem biologischen Gewebe, das seinerseits ebenfalls mit dem Generator verbunden sein kann. Die Verbindung des Gewebes mit dem Generator kann beispielsweise über eine am Patienten angebrachte Neutralelektrode, eine in das Instrument integrierte zweite Elektrode, eine weitere Plasmastrecke oder über die kapazitive Kopplung des Generators und des Patienten mit dem Erdpotential erfolgen. Der Behandlungsstrom kann auch als Verschiebungsstrom unter Ausnutzung der Kapazität des Gewebes fließen.

Das Instrument kann einen Gasführungskanal aufweisen, in oder an dem die Elektrode angeordnet ist. Der Gasführungskanal ist an seinem proximalen Ende an eine Gasquelle, beispielsweise eine Argonquelle, angeschlossen, so dass an seinem distalen Ende ein Gasstrahl ausströmen kann, in dem durch Ionisation ein elektrisch leitfähiges Plasma z.B. in Form eines Funkens generiert werden kann.

Vorzugsweise ist die Hochfrequenzspannung mit einer Mittelfrequenz gepulst, die zum Beispiel kleiner als ein Fünftel der Frequenz der Hochfrequenzspannung, jedoch vorzugsweise zum Beispiel größer als ein Zwanzigstel derselben ist. Die Frequenz der Hochfrequenzspannung liegt zum Beispiel in einem Frequenzbereich zwischen 100 kHz und 20 MHz und beträgt beispielsweise 350 kHz. Die Mittelfrequenz liegt beispielsweise im Bereich von 10 kHz bis 100 kHz. Sie kann zum Beispiel 20 kHz, 30 kHz, 45 kHz oder 50 kHz betragen. Die für den Generator zur Verfügung stehenden von der Steuereinrichtung auswählbaren Spannungsformen können sich zum Beispiel bei festgelegter Frequenz von 45 kHz oder 50 kHz durch die Pulslänge unterscheiden, wobei das Sortiment der verfügbaren Spannungsformen auch Pulsfolgen mit niedrigerer oder höherer Frequenz enthalten kann.

Zusätzlich kann die mit der Mittelfrequenz gepulste Hochfrequenzspannung mit einer Niederfrequenz gepulst sein, die vorzugsweise mindestens 0,5 Hz, weiter vorzugsweise höchstens aber 200 bis maximal 500 Hz beträgt. Ein bevorzugter Frequenzbereich der Niederfrequenz geht von 1 Hz bis 100 Hz. Die Spannungsformen der Hochfrequenzspannung können sich bedarfsweise auch durch verschiedene Pulslängen und/oder verschiedenen Pausenlängen der Niederfrequenz unterscheiden. Die Pausenlänge kann auch Null sein.

Der Plasmasensor dient der Feststellung des Verhaltens eines Plasmas an dem Behandlungsinstrument. Der Plasmasensor kann wie auch der Generator und eine Steuereinrichtung Bestandteil eines Geräts zur Versorgung des Instruments sein. Der Plasmasensor erfasst mindestens eine das Vorhandensein oder Nichtvorhandensein und/oder die Qualität bzw. Stabilität des Plasmas kennzeichnende Größe. Das kann beispielsweise auf indirektem Wege erfolgen, indem der Plasmasensor dazu eingerichtet ist, die zu dem Instrument übertragene Spannung und/oder den zu dem Instrument fließenden Strom zu erfassen und diese Größen oder daraus abgeleitete Größen zu überwachen. Zum Beispiel kann der Plasmasensor aus der gemessenen Spannung und dem gemessenen Strom auf eine an dem Instrument wirksame Impedanz schließen, die sich im Wesentlichen aus der Impedanz des Plasmas und der Impedanz des biologischen Gewebes zusammensetzt. Diese Impedanz und/oder deren zeitliche Veränderung können als Indikator für die Plasmastabilität und/oder -qualität genutzt werden. In weiteren Ausführungsformen können andere Größen und/oder deren zeitliche Veränderung als Maß für die Qualität bzw. die Stabilität des Plasmas genutzt werden, die durch geeignete Sensoren gemessen und/oder aus Messwerten abgeleitet werden können. Solche Größen sind beispielsweise der elektrische Strom, die elektrische Leistung, weitere gemessene oder berechnete Eigenschaften des elektrischem Stroms, der elektrischen Spannung und/oder einer Kombination daraus, die Intensität und/oder das Spektrum einer optischen Leuchterscheinung, die Temperatur des Gases bzw. Plasmas, das elektrische und/oder magnetische Feld und/oder die elektromagnetische Strahlung des Plasmas. Diese oder andere geeignete Größen können wie oben beschrieben einzeln oder in Kombination herangezogen werden, um Plasmastabilität und/oder -qualität zu ermitteln.

Die an dem Plasmasensor angeschlossene Steuereinrichtung ist darauf eingerichtet, die Generatoreinstellung anhand des von dem Plasmasensor erfassten Verhaltens des Plasmas auszuwählen und den Generator zu veranlassen, eine Spannung mit bestimmten Spannungseigenschaften abzugeben. Dazu kann die Steuereinrichtung weiter darauf eingerichtet sein, die Generatoreinstellung bei einer Aktivierung des Behandlungsinstruments anhand gelegentlicher oder fortwährender Tests festzulegen. Dazu kann die Steuereinrichtung darauf eingerichtet sein, bei einer gegebenen Generatoreinstellung zunächst das Zündverhalten und/oder das Plasmaerhaltungsverhalten zu überprüfen und, falls das Zünd- oder Plasmaerhaltungsverhalten nicht ausreichend ist, eine Generatoreinstellung mit besserer Zündfähigkeit und/oder Plasmaerhaltungsfähigkeit zu wählen. Weiter kann die Steuereinrichtung darauf eingerichtet sein, wenn sie mindestens einmal, vorzugsweise mehrmals, ein sicheres schnelles Zünden und/oder ein sicheres Halten des Plasmas festgestellt hat, eine Generatoreinstellung mit niedrigerer Zündfähigkeit und/oder Plasmaerhaltungsfähigkeit zu wählen und den Generator zu veranlassen, zu einer solchen überzugehen. Es kann vorgesehen sein, dass diese Generatoreinstellung zunächst beibehalten wird, wenn damit sicherer Betrieb möglich ist. Falls nicht, wird zu der vorigen oder einer anderen Generatoreinstellung mit besserer Zündfähigkeit und/oder besserer Plasmaerhaltung gewechselt.

Mit dieser Einrichtung wird einerseits ein sicheres Zünden und Erhalten des Plasmas beim Koagulieren ermöglicht, ohne dazu mit einem zumindest temporären Überschuss an Leistung, Spitzenspannung, Frequenz oder einer oder mehreren anderen, die Spannungsform der betreffenden Generatoreinstellung kennzeichnenden Größen arbeiten zu müssen. Unter einem solchen Überschuss wird eine Generatoreinstellung verstanden, bei der die Zündfähigkeit und/oder die Plasmaerhaltungsfähigkeit der Hochfrequenzspannung größer als nötig ist. Durch Zündung des Plasmas ohne (oder mit lediglich kleinem, bestenfalls kleinstmöglichem) Überschuss können andererseits unerwünschte Nebeneffekte der Plasmazündung und/oder des durch das Plasma fließenden Stroms weitgehend unterdrückt werden, so dass das Gewebe und /oder das Instrument nicht (mehr als nötig) geschädigt, der Behandler weder geblendet noch erschreckt wird. Solche unerwünschten Nebeneffekte können beispielsweise die Ausbildung sehr heller Lichterscheinungen oder die Erzeugung von Knallgeräuschen, unerwünschte Gewebeschädigungen, Instrumentenschädigungen oder dergleichen mehr sein. Durch die schrittweise Annäherung der Einstellung des Generators an eine niedrigst mögliche Einstellung (d.h. eine Einstellung, die gerade noch eine Zündung und/oder einen stabilen Plasmaerhalt ermöglicht) können unerwünschte Nebeneffekte minimiert werden. Dagegen können aber erwünschte Effekte der Plasmazündung und/oder des durch das Plasma fließenden Stroms maximiert oder anderweitig gezielt beeinflusst werden, beispielsweise durch eine entsprechende Auswahl der zur Verfügung stehenden Einstellungen.

Zu Beginn der Aktivierung des Instruments sowie ggf. zu Beginn jedes Pulses der niederfrequenten Frequenz muss jeweils ein Plasma gezündet werden. In jedem Puls der niederen Frequenz kann eine mittelfrequente Folge von Hochfrequenzpulsen enthalten sein. Die Steuereinrichtung ist vorzugsweise derart ausgebildet, dass sie während des Betriebs des Instruments, d.h. während der Beaufschlagung desselben mit verschiedenen hochfrequenten Spannungsformen, den Generator mit derjenigen Einstellung betreibt, die bei minimaler Zündfähigkeit der Spannungsform innerhalb einer Zündversuchszeit zu einer Erzeugung von Plasma führt. Damit wird eine Einstellung verwendet, welche mindestens eine das Zünden des Plasmas beeinflussende Eigenschaft, z.B. das Puls/Pause-Verhältnis, gerade im nötigen Maß, nicht jedoch einen Überschuss aufweist. Dadurch werden die mit der Zündung des Plasmas einhergehenden unerwünschten Auswirkungen minimiert, d.h. Auswirkungen der Zündung werden in gewünschter Weise beeinflusst.

Vorzugsweise ist die Steuereinrichtung derart ausgebildet, dass sie während des Betriebs des Instruments nach Ablauf der Zündversuchszeit den Generator mit einer Einstellung mit verbesserter Zündfähigkeit, z.B. durch ein vergrößertes Puls/Pause-Verhältnis, weiterbetreibt, wenn mittels des Plasmasensors innerhalb der Zündversuchszeit keine Erzeugung von Plasma erfasst worden ist. Damit wird erreicht, dass das Zünden des Plasmas mit einer Spannungsform bewirkt wird, die gerade eben zündfähig genug ist, um ein sicheres Zünden zu bewirken, jedoch nicht unnötig zündfähiger.

Eine ähnliche Strategie wie für das Zünden des Plasmas kann für dessen Erhalt während der Behandlungsphase angewandt werden. Dazu ist die Steuereinrichtung derart ausgebildet, dass sie während des Betriebs des Systems den Generator nach Ablauf einer Zündphase in einer Behandlungsphase mit derjenigen Einstellung betreibt, die bei minimaler Plasmaerhaltungsfähigkeit der Spannungsform den Erhalt des Plasmas gestattet. Mit anderen Worten, sie wählt diejenige Einstellung für den Betrieb in der Behandlungsphase, die einerseits den Plasmaerhalt sicher stellt und andererseits unter den Einstellungen des Generators diejenige ist, bei der ein ausgewählter Nebeneffekt möglichst gering ist. Der Nebeneffekt ist z.B. die Lichterscheinung, die Schallentwicklung, die Gewebepunktierungsneigung, die Plasmatemperatur, insbesondere dessen Ionentemperatur, oder dergleichen mehr. Die gleiche Steuerstrategie wird für die Zündphase angewendet.

Dazu kann die Steuereinrichtung so ausgebildet sein, dass sie während des Betriebs des Instruments, nach erfolgtem Aufbau des Plasmas, den Generator mit einer gegebenen Einstellung betreibt und dabei die Plasmastabilität überwacht. Wenn ein drohendes Verlöschen oder gar ein Verlöschen des Plasmas erfasst wird, wechselt die Steuereinrichtung zu einer Einstellung mit einer besseren Plasmaerhaltung, z.B. durch ein größeres Puls/Pause-Verhältnis der Hochfrequenzspannung. Alternativ oder zusätzlich kann die Steuereinrichtung, wenn das Plasma abgerissen oder instabil geworden ist, nach der nächsten Zündphase eine Einstellung vorgeben, die eine z.B. aufgrund eines größeren Puls/Pause-Verhältnisses der Hochfrequenzspannung bessere Plasmastabilität erbringt. Wurde hingegen bei der Behandlung mit einer gegebenen Spannungsform während einer bestimmten Zeit kein drohendes Verlöschen, sondern stabiler Plasmaerhalt festgestellt, kann die Steuereinrichtung versuchsweise eine Einstellung mit schlechterer Plasmaerhaltungsfähigkeit, z.B. durch ein geringeres Puls/Pause-Verhältnis, wählen, um Licht- und Schallerscheinung, Leistungseintrag, Gewebeeffekt oder andere Auswirkungen des Plasmastroms innerhalb der Behandlungsphase zu minimieren und/oder in die gewünschte Richtung zu beeinflussen.

Durch die Festlegung einer Einstellung des Generators, bei der die Mittelfrequenz ein minimales Puls/Pause-Verhältnis, die Hochfrequenzspannung eine minimale Spitzenspannung oder eine andere, den Leistungseintrag beeinflussende Eigenschaft aufweist, wird der Leistungseintrag während der Behandlungsphase eines Pulses der Niederfrequenz beeinflusst, insbesondere minimiert. Um jedoch einen gewünschten mittleren Leistungseintrag realisieren zu können, kann die Steuereinrichtung weiterhin so ausgebildet sein, dass sie das Puls/Pause-Verhältnis der Niederfrequenz oder ein anderes, den Leistungseintrag beeinflussendes Merkmal der Spannungsform so einrichtet, dass die tatsächlich an das Instrument gelieferte mittlere Leistung einem Sollwert entspricht. Wenn die Steuereinrichtung eine Spannungsform mit größerem Leistungseintrag während des Pulses der Niederfrequenz wählt, kann sie den dadurch erhöhten Leistungseintrag wieder reduzieren, d.h. ausgleichen, indem sie z.B. das Puls/Pause-Verhältnis der Niederfrequenz reduziert und umgekehrt. Dadurch ist es möglich, verschiedene Betriebsarten bereitzustellen, die unterschiedliche Zündfähigkeiten und/oder Plasmaerhaltungsfähigkeiten, dabei aber gleichen Leistungseintrag aufweisen.

Das erfindungsgemäße Verfahren kann z.B. so eingerichtet sein, dass die HF-Leistung während eines Pulses einer niederfrequent gepulsten HF-Wechselspannung begrenzt oder minimiert wird. Jeder niederfrequente Puls enthält dann eine mittelfrequente Folge von hochfrequenten Schwingungen. Die Leistung des niederfrequenten Pulses wird z.B. niedrigst möglich eingestellt. Durch Anpassung des niederfrequenten Puls/Pause-Verhältnisses kann dennoch im Mittel die gewünschte Sollleistung bereitgestellt werden. Die Behandlung verläuft mit geringer Licht- und Lärmentwicklung.

Weitere Einzelheiten vorteilhafter Ausführungsformen der Erfindung sind Gegenstand der Unteransprüche, der Zeichnung sowie der zugehörigen Beschreibung. In der Zeichnung zeigen:
Figur 1 eine Behandlungseinrichtung, in sehr stark schematisierter Darstellung,
Figur 2 eine niederfrequente Hüllkurve einer mittelfrequent gepulsten Hochfrequenzspannung zur Verdeutlichung des Betrieb der Einrichtung nach Figur 1,
Figur 3 einen Puls der Pulsfolge nach Figur 2, in gedehnter Darstellung,
Figur 3A bis 3D verschiedene Spannungsformen der mittelfrequent gepulsten hochfrequenten Generatorschwingung bei verschiedenen Puls/Pause-Verhältnissen innerhalb eines Pulses nach Figur 3 als Spannungsformen für Zündbetrieb und /oder Behandlungsbetrieb,
Figur 4 einen beispielhaften Ablaufplan zur geeigneten Auswahl einer Spannungsform für den Zündbetrieb zur Verdeutlichung der Funktion der Steuereinrichtung,

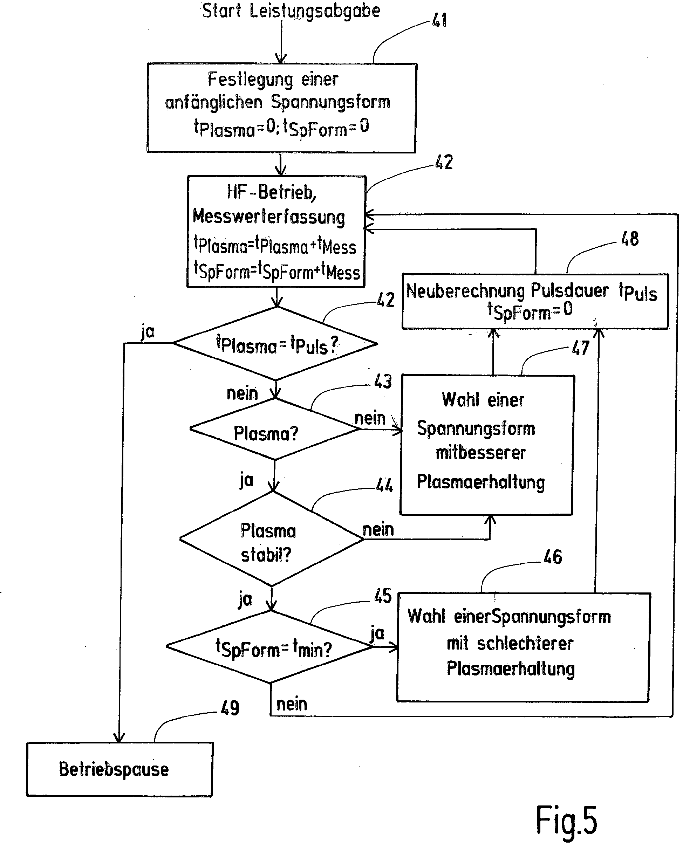

Figur 5 einen beispielhaften Ablaufplan zur Verdeutlichung des Betriebs der Steuereinrichtung im Behandlungsbetrieb.

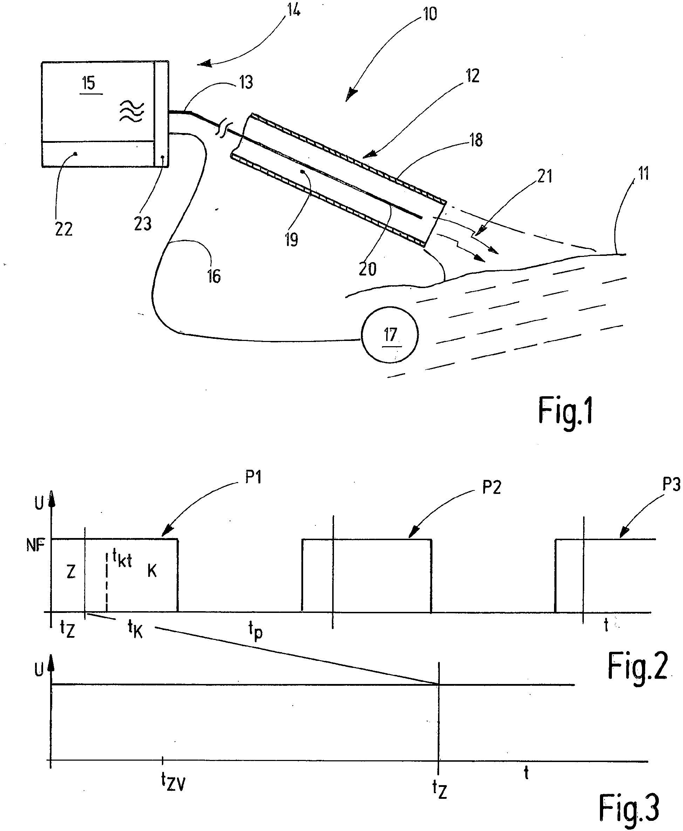

Figur 1 veranschaulicht eine Einrichtung 10 zur Plasmabehandlung von biologischem Gewebe 11, beispielsweise bei einem chirurgischen Eingriff. Zu der Einrichtung 10 gehört ein Instrument 12, beispielsweise in Gestalt eines offenchirurgisch zu verwendenden Instruments oder in Gestalt einer starren oder flexiblen, zum Beispiel in einem Endoskop einzusetzenden Sonde. Zur Speisung des Instruments 12 ist dieses über eine Leitung 13 mit einem Gerät 14 verbunden, das einen Generator 15 zur Versorgung des Instruments 12 mit hochfrequentem Strom enthält.

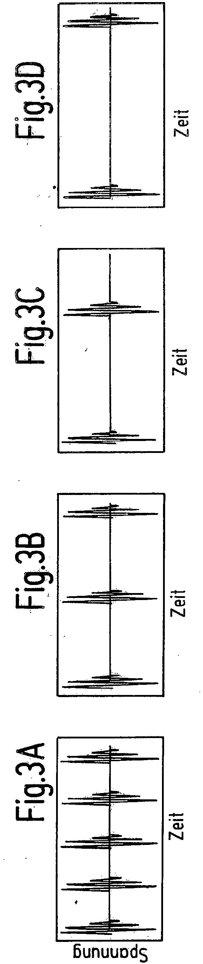

Der Generator 15 ist derart ausgebildet, dass er bei Aktivierung des Instruments 12 eine Hochfrequenzspannung an das Instrument 12 abgibt, wobei er in unterschiedlichen Einstellungen arbeiten kann. In jeder der Einstellungen unterscheidet sich der Generator in wenigstens einer elektrischen Eigenschaft von allen anderen Einstellungen. Eine elektrische Eigenschaft kann die Form, Frequenz, Modulationsart, der Crest-Faktor, das Puls/Pause-Verhältnis einer erzeugten gepulsten Hochfrequenzspannung, die Größe der Hochfrequenzspannung, der Innenwiderstand des Generators, der Maximalstrom oder ähnliches sein. Die Figuren 3A bis 3D illustrieren dies am Beispiel verschiedener Puls/Pause-Verhältnisse einer gepulsten Hochfrequenzspannung.

Das Gerät 14 kann außerdem über eine Neutralleitung 16 mit einer Neutralelektrode 17 verbunden sein, die an dem biologischen Gewebe 11, beispielsweise an der Haut eines Patienten, großflächig angebracht ist. Alternativ kann das Instrument 12 als bipolares Instrument ausgebildet sein und dazu mindestens zwei Elektroden aufweisen.

Das Instrument 12 enthält einen durch ein starres Rohr oder einen flexiblen Schlauch 18 gebildeten Kanal 19 sowie eine Elektrode 20, die kurz vor, an oder hinter dem distalen Ende des Schlauchs 18 endet, um ein zu dem Gewebe 11 überspringendes Plasma21 zu erzeugen. Der Kanal 19 ist an eine nicht weiter veranschaulichte Gasquelle angeschlossen und somit von einem Gas, beispielsweise Argon, durchströmt, das an dem distalen Ende des Schlauchs 18 austritt und in dem sich das Plasma 21 zwischen Elektrode 20 und Gewebe 11 bildet. Die Erfindung kann aber auch bei Instrumenten ohne Gaszuführung angewendet werden.

Das Gerät 14 enthält neben dem Generator 15 eine Steuereinrichtung 22, die die Betriebsart des Generators 15 festlegend ausgebildet ist und über die Generator 15 ein- und ausschaltbar ist. Die Steuereinrichtung 22 ist z.B. dazu eingerichtet, dem Generator 15 die von ihm zu liefernde Spannungsform vorzugeben.

Außerdem enthält das Gerät 14 einen Plasmasensor 23, der die Spannung zwischen der Leitung 14 und der Neutralleitung 16 sowie den in der Leitung 13 und/oder der Leitung 16 fließenden Strom erfasst und daraus eine das Vorhandensein oder Nichtvorhandensein des Plasmas sowie die Qualität desselben kennzeichnende Größe ableitet. Im einfachsten Fall ist diese Größe der erfasste Strom selbst. Es ist aber auch möglich, den Plasmasensor so auszubilden, dass er aus der erfassten Spannung und/oder dem erfassten Strom abgeleitete Größen zur Kennzeichnung der Plasmaqualität ableitet. Solche Größen können beispielsweise die Größe des fließenden Stroms, die Geschwindigkeit der Änderung des Stroms, die als Quotient zwischen der erfassten Spannung und dem erfassten Strom errechnete Impedanz, die Änderung der Impedanz (Impedanzanstieg und/oder Impedanzabfall), die Geschwindigkeit der Änderung der Impedanz, Impedanzfluktuationen, Stromverzerrungsfaktoren, Oberwellengehalt des Stroms, Crest-Faktor des Stroms, die Differenz zwischen dem Oberwellengehalt des Stroms und dem Oberwellengehalt der Hochfrequenzspannung oder ähnliche Größen sein.

Zur Plasmabehandlung wird das Behandlungsinstrument 12 vorzugsweise mit einer hochfrequenten, von dem Generator 15 erzeugten Hochfrequenzspannung HF beaufschlagt, die vorzugsweise eine Frequenz zwischen 100 kHz und 20 MHz hat und im vorliegenden Ausführungsbeispiel 350 kHz beträgt. Die Hochfrequenzspannung HF ist typischerweise mit einer Mittelfrequenz MF und zusätzlich mit einer Niederfrequenz NF gepulst. Die entstehenden, niederfrequent aufeinander folgenden Pulspakete P1, P2, P3 können eine Dauer von 2 ms bis zu zwei Sekunden aufweisen. Zwischen den Pulspaketen P1, P2, P3 sind Pausen von, je nach Puls/Pause-Verhältnis, fast zwei Sekunden bis zu nahezu Null Sekunden. Innerhalb der Pulspakete P1, P2, P3 stellt der Generator 15 die Hochfrequenzspannung HF mit verschiedenen Spannungsformen bereit. Eigenschaften derselben können an dem Generator 15 und/oder der Steuereinrichtung 22 über eine gegebenenfalls vorhandene Bedienschnittstelle und/oder eine Kommunikationsschnittstelle zur Verbindung mit weiteren Geräten, beispielsweise mobilen Endgeräten eingestellt der eingegrenzt werden. Dadurch können behandlungsbeeinflussende Parameter, wie zum Beispiel Spitzenwert der Hochfrequenzspannung, maximaler Strom, gewünschte Leistung, Behandlungsdauer oder Ähnliches, einstellbar sein. Zudem können bedarfsweise Mittel zur Auswahl oder Eingrenzung wählbarer Einstellungen des Generators vorgesehen sein, aus denen die Steuereinrichtung 22 eine für den Betrieb des Generators 15 geeignete Einstellung und die sich daraus ergebende Spannungsform auswählen kann.

Das in Figur 2 beispielhaft veranschaulichte Pulspaket P1 der niederfrequent aufeinander folgenden Pulspakete P1, P2, P3 ist in Figur 3 vergrößert veranschaulicht. Es unterteilt sich in eine Zündphase Z und eine Behandlungsphase K. Die Zündphase Z dient zum Aufbau eines Plasmas und zur stabilen Ionisation desselben. Die dafür bereitstehende Zeit t_{Z} kann, wie Figur 3 veranschaulicht, eine Zündversuchszeit t_{ZV} enthalten, die zum Beispiel eine festgelegte Zeitspanne von zum Beispiel 1 ms oder ein festgelegter Bruchteil der Zündzeit t_{Z} sein kann. Innerhalb dieser Zündversuchszeit t_{ZV} wird überwacht, ob bei der anliegenden Hochfrequenzspannung ein Zünden des Plasmas bzw. des Funkens eingetreten ist.

Während des Zündens, d.h. mindestens bis zum Ablauf der Zündversuchszeit t_{ZV}, arbeitet der Generator 15, gesteuert von der Steuereinrichtung 22, mit einer anfänglichen Einstellung, die aus mehreren möglichen vorbestimmten Einstellungen ausgewählt ist. Die Figuren 3A bis 3D zeigen die Spannungsformen der betreffenden Einstellungen, die vorzugsweise übereinstimmende HF-Frequenz und Spitzenspannung aufweisen. Sie sind mit einer mittelfrequenten Modulationsspannung gepulst. Beispielsweise beträgt Pulsfrequenz irgendeine Frequenz zwischen 10 kHz und 70 kHz. Nach einer oder mehreren ungedämpften Schwingungen schwingt die Hochfrequenzspannung aus, d.h., sie klingt gedämpft ab. Figur 3A veranschaulicht eine Einstellung mit einem großen PulsPause-Verhältnis. Die mittelfrequente Modulationsfrequenz beträgt hier 50 kHz. Diese Spannungsform weist unter den zur Auswahl stehenden Spannungsformen die besten Zündeigenschaften, aber auch die stärkste Leucht- und Geräuschentwicklung auf.

Figur 3B veranschaulicht eine zweite Einstellung, bei der die mittelfrequente Modulationsfrequenz auf 40 kHz festgelegt ist. Diese Spannungsform weist gute Zündeigenschaften bei abgeschwächter Licht- und abgeschwächter Geräuschentwicklung auf.

Figur 3C veranschaulicht eine dritte Einstellung, bei der die Hochfrequenzspannung HF mit einer Mittelfrequenz MF von 30 kHz moduliert ist, wodurch das Puls/Pause-Verhältnis gegenüber Figur 3B weiter verringert ist. Bei etwas verschlechterten Zündeigenschaften ist die Licht- und Geräuschentwicklung weiter reduziert.

Figur 3D veranschaulicht eine weitere Einstellung, die die Steuereinrichtung 22 gegebenenfalls auswählen und der Generator 15 bereitstellen kann. Das Tastverhältnis und die Frequenz der Mittelfrequenz MF sind erneut reduziert. Sie liegt noch immer in dem angegebenen Bereich von 10 bis 70 kHz und beträgt hier beispielsweise 20 kHz.

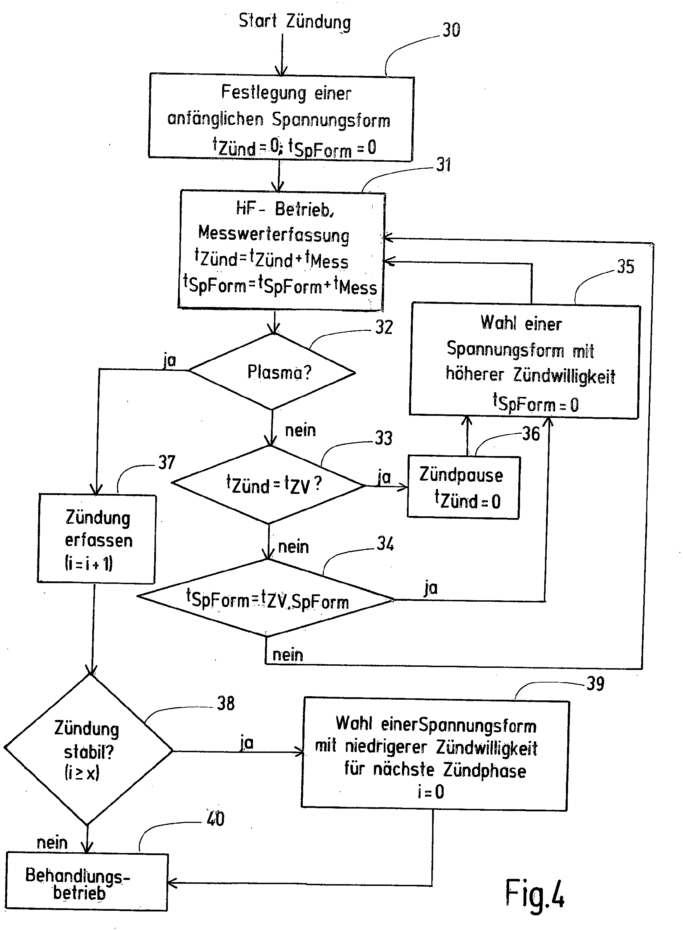

In der Zündphase Z arbeitet die Einrichtung 10 wie folgt:
Die Steuereinrichtung 22 wählt zum Zünden eines Plasmaszu Beginn des Pulspakets P1 eine der genannten oben erläuterten vier Einstellungen nach Figur 3A bis 3D aus. Dieser Vorgang ist in Figur 4 in Block 30 veranschaulicht. Zu Behandlungsbeginn wählt die Steuereinrichtung 22 die Einstellung, beispielsweise gemäß manueller Vorgabe oder entsprechend einer zuletzt genutzten Einstellung aus. Die im weiteren Verlauf benötigten Zeitzähler t_{Zünd} und t_{SpForm} werden inital auf Null gesetzt. Sodann beginnt der Generator 15 die Erzeugung der Hochfrequenzspannung (siehe Block 31). Innerhalb eines Zeitraumes t_{Mess} werden die Messwerte, beispielsweise Strom, Spannung und andere Größen, erfasst und abgeleitete Größen, beispielsweise der Wert des Plasmasensors 23, berechnet. Die Zeitzähler werden jeweils um t_{Mess} erhöht. Anschließend wird überprüft, ob ein Plasma erzeugt worden ist oder nicht (Block 32). Ist dies nicht der Fall, wird überprüft, ob die Zündversuchszeit t_{ZV} abgelaufen ist (Block 33). Ist dies der Fall, wird der Zündversuch beendet und eine Zündpause eingeschoben (Block 36). Falls nicht, wird überprüft, ob die für eine Spannungsform vorgesehene Zündversuchszeit t_{ZV_SpForm}, die kürzer ist als t_{ZV} (zum Beispiel ein Viertel der zur Verfügung stehenden Zündzeit t_{ZV} oder eine festgelegte Zeit von zum Beispiel einer Millisekunde), abgelaufen ist (Block 34). Ist eine der beiden Zündversuchzeiten abgelaufen, ohne dass ein Plasma gezündet hat, wählt die Steuereinrichtung eine Spannungsform mit höherer Zündfähigkeit, wie Block 35 veranschaulicht. Gleichzeitig wird die Zeitzählung t_{Sp_Form} für die Spannungsform zurückgesetzt. Hatte die Steuereinrichtung 22 zu Beginn zum Beispiel die Spannungsform gemäß Figur 3C vorgegeben, wechselt sie nun auf die Spannungsform gemäß Figur 3B. Es wird nun gemäß Block 31 wiederum eine Messzeit durchlaufen und danach überprüft, ob ein Plasma gezündet hat. Falls dies bis zum Ablauf einer der Zündversuchszeiten nicht der Fall ist, wird in Block 35 wiederum eine Spannungsform mit noch höherer Zündfähigkeit gewählt, beispielsweise die Spannungsform nach Figur 3A.

Hat ein Plasma gezündet, wird die erfolgreiche Zündung registriert, z.B. indem in Block 37 eine Zählvariable i inkrementiert wird. In Block 38 wird überprüft, ob die erfasste Anzahl X erfolgreicher Zündversuche erreicht worden ist. Ist dieses und/oder ein anderes Stabilitätskriterium erfüllt, wechselt die Steuereinrichtung 22 in Block 39 unter Rücksetzung der Zählvariable i auf Null für die nächste Zündphase der Niederfrequenz versuchsweise zu einer Spannungsform mit niedrigerer Zündfähigkeit, um zu testen, ob sich die Zündung auch mit niedrigerer Licht- und Geräuschentwicklung bewirken lässt. Anschließend wird direkt zum Behandlungsbetrieb übergegangen (Block 40).

An den Zündbetrieb schließt sich der Behandlungsbetrieb K an, für den wiederum verschiedene Einstellungen gemäß Figur 3A bis 3D bereitstehen. Die Spannungsform gemäß Fig. 3A hat die besten Plasmaerhaltungseigenschaften, die größte mittlere Leistung und die stärkste Licht- und Geräuschentwicklung. Die Spannungsformen gemäß Fig. 3B, 3C und 3D haben von links nach rechts jeweils eine schlechtere Plasmaerhaltungsfähigkeit, eine kleinere mittlere Leistung und eine geringere Licht- und Geräuschentwicklung als die in der Reihenfolge vorhergehende Spannungsform.

Für die Behandlungsphase kann wie in diesem Beispiel die gleiche, aber auch eine andere Auswahl von Spannungsformen verwendet werden als für die Zündphase.

In der Behandlungsphase K arbeitet die Einrichtung 10 vorzugsweise wie folgt:

Zu Beginn der Behandlungsphase wird, wie Block 41 symbolisiert, mit einer anfänglichen Spannungsform gearbeitet. Diese kann zum Beispiel vorgegeben oder aus der Historie abgeleitet sein und der zuletzt erfolgreich genutzten Spannungsform entsprechen. Die im weiteren Verlauf benötigten Zeitzähler t_{Plasma} und t_{SpForm} werden inital auf Null gesetzt. Die Spannungsform wird an das Instrument 12 geliefert, wie Block 42 symbolisiert. Innerhalb eines Zeitraumes t_{Mess} werden wie in der Zündphase Messwerte erfasst und abgeleitete Größen, beispielsweise der Wert des Plasmasensors 23, berechnet. Die Zeitzähler werden jeweils um t_{Mess} erhöht. Anschließend wird der Zeitzähler t_{Plasma} mit einer durch die eingestellte Leistungsabgabe gegebenen Zeit t_{Puls} verglichen (Block 42). Ist die Zeit t_{Puls} verstrichen, erfolgt ein Wechsel zur Betriebspause (Block 49). Ansonsten wird anhand des Plasmasensors der Zustand des Plasmas abgefragt. Bei einer bevorzugten Ausführungsform ist der Plasmasensor 23 darauf eingerichtet, nicht nur eine Unterbrechung des Plasmas (Block 43), sondern auch eine sich ankündigende Unterbrechung des Plasmas (Block 44) zu erfassen. Dazu kann er zum Beispiel darauf eingerichtet sein, die zwischen den Leitungen 13 und 16 zu messende Impedanz und deren zeitlichen Verlauf zu überwachen. Steigt die Impedanz innerhalb von einer gegebenen Zeitspanne von zum Beispiel 200 µs um mehr als beispielsweise 10% oder einen festgelegten Wert von zum Beispiel 200 Ohm an, ist dies ein Indiz für eine bevorstehende Plasmaunterbrechung. Der Plasmasensor 23 kann darauf eingerichtet sein, dies zu erfassen. Die Steuereinrichtung 22 kann außerdem darauf eingerichtet sein, bei Vorliegen eines Anzeichens für bevorstehende Plasmaunterbrechung gemäß Block 44 sofort eine Einstellung mit besseren Plasmaerhaltungseigenschaften auszuwählen (Block 47). Zum Beispiel geht sie von einer Spannungsform gemäß Figur 3D auf eine Spannungsform gemäß Figur 3C, von einer Spannungsform nach Figur 3C auf eine Spannungsform nach Figur 3B oder von einer Spannungsform nach Figur 3B auf eine Spannungsform nach Figur 3A über. Letztendlich springt die Steuereinrichtung 22 in Block 44 in der Reihe der Spannungsformen nach Figur 3A bis 3D jeweils um mindestens eine Stufe nach links.

Ist das Plasma während einer Zeitdauer von mindestens tₘᵢₙ stabil, wählt die Steuereinrichtung testweise eine Spannungsform mit schlechteren Plasmaerhaltungseigenschaften, indem sie in der Reihe der Spannungsformen nach Figur 3A bis 3D um mindestens eine Stufe nach rechts springt. Die Zeit tₘᵢₙ kann hierbei für jede Spannungsform individuell festgelegt werden. Ist die Behandlung während der Zeit tₘᵢₙ beispielsweise erfolgreich mit der Spannungsform nach Figur 3B durchgeführt worden, wechselt die Steuereinrichtung zu der Spannungsform nach Figur 3C. Sofern sich eine Plasmainstabilität abzeichnen sollte, wird auf die Spannungsform nach Figur 3B zurück gewechselt. Bleibt das Plasma jedoch mit der Spannungsform nach Figur 3C stabil erhalten, ist die Leucht- und Geräuscherscheinung minimiert.

Auf die hier vorgestellte Weise wählt die Steuereinrichtung 22 jeweils die Spannungsform für das Zünden und die Behandlung, die möglichst geringen unerwünschten Nebeneffekten, wie Licht- und Geräuschentwicklung, einhergeht. Die Steuereinrichtung kann eine oder mehrere andere Auswirkungen des Zündens und/oder Plasmastroms in die gewünschte Richtung beeinflussen, wobei andererseits sicheres Zünden und sichere Plasmabehandlung erreicht wird. Durch den Wechsel der Einstellungen des Generators 22 während des Zündens und/oder während der Behandlung innerhalb der und zwischen den einzelnen Pulspaketen P1, P2, P3 kann die auf das biologische Gewebe 11 übertragene Leistung beeinflusst werden. Die Steuereinrichtung 22 hat das Minimierungsziel in den Figuren 3A bis 3D jeweils eine möglichst weit rechts liegende Spannungsform, d.h. eine Spannungsform mit einem Tastverhältnis zu wählen, das so gering wie möglich ist. Dies geht mit vermindertem Leistungseintrag einher.

Um jedoch den gewünschten mittleren Leistungseintrag Pₛₒₗₗ sicherzustellen, kann die Steuereinrichtung 22 darauf eingerichtet sein, zum Ausgleich die Dauer der Pulspakete P1, P2, P3 zu verlängern oder zu verkürzen, wie es in Figur 6 durch Block 48 veranschaulicht ist. Dazu kann die Steuereinrichtung 22 darauf eingerichtet sein, die in einem Pulspaket bisher an das biologische Gewebe übertragene Energiezu erfassen und die Zeit t_{Puls} so zu berechnen, dass bei Beibehaltung der Spannungsform nach Ablauf der Zeit t_{Puls} die übertragene Energie dem Produkt aus Leistungssollwert Pₛₒₗₗ und Gesamtzeit t_{G} entspricht. Die Gesamtzeit t_{G} ist die Summe aus Zündzeit t_{Z}, Behandlungszeit t_{K} und Pausenzeit t_{P} und beträgt bei festgelegter Niederfrequenz von zum Beispiel 50 Hz 20 ms.

Es sind Abwandlungen der Behandlungsphase möglich. Zum Beispiel kann die Steuereinrichtung nach Ablauf einer bestimmten Zeit, zum Beispiel 0,5 ms oder einen Anteil der vorgesehenen Dauer des Pulspakets, überprüfen, ob immer noch ein Plasma vorhanden ist und, falls dies der Fall ist, zu einer Spannungsform mit schlechteren Plasmaerhaltungseigenschaften, dafür aber geringerer Geräusch- und Leuchtentwicklung wechseln. Wird dabei das Plasma unterbrochen oder zeigt es danach mangelnde Stabilität, kann innerhalb der gleichen Behandlungsphase von der Steuereinrichtung 22 ein Wechsel zurück zu einer Spannungsform mit besseren Erhaltungseigenschaften veranlasst werden.

Unabhängig davon kann bei allen vorgestellten Ausführungsformen bei jedem Wechsel der Spannungsform die Dauer des Pulspakets P1, P2 und/oder P3 entsprechend den Eigenschaften der bisher und aktuell verwendeten Spannungsform angepasst werden, um die ins Gewebe eingebrachte mittlere Leistung P möglichst konstant zu halten.

Die Auswahl der Einstellungen für die Zünd- und Behandlungsphase kann in jedem Pulspaket so ausgewählt werden, wie es in der vorausgegangenen Zünd- und Behandlungsphase zuletzt verwendet wurde. Beispielsweise kann das Pulspaket P2 mit der Einstellung starten, mit der das Zünden zuletzt in dem Pulspaket P1 bewirkt worden ist. Entsprechendes gilt für die Spannungsform zur Behandlung.

Während gemäß der vorstehenden Beschreibung ein Wechsel zwischen den verschiedenen Spannungsformen nach den Figuren 3A bis 3D im Sinne eines Umschaltens ausgegangen worden ist, kann dieser Wechsel bedarfsweise auch gleitend oder stufenlos erfolgen.

Bei einem abgewandelten Ausführungsbeispiel des Geräts 14 ist die Steuereinrichtung 22 darauf eingerichtet, die von dem Plasmasensor 23 gemessene Impedanz darauf zu überprüfen, ob diese innerhalb eines vorgegebenen Wertebereichs liegt, zum Beispiel zwischen 100 Ohm und 9,5 kOhm. Ist dies der Fall, kann die Steuereinrichtung 22 das Vorliegen eines Plasmas annehmen. Alternativ kann die Größe des gemessenen Effektivstroms als Indikator vorgesehen sein. In diesem Fall ist der Plasmasensor 23 darauf eingerichtet, zu erfassen, ob der gemessene Effektivstrom größer als ein vorgegebener Wert von zum Beispiel 0,1 Ampere ist. Zusätzlich kann der Plasmasensor 23 darauf eingerichtet sein, die Impedanz zu überwachen. Ist diese kleiner als ein vorgegebener Wert von zum Beispiel 3 kOhm, kann dies in Kombination mit der vorgenannten Schwellwertüberschreitung des Stroms als Indikator für das Vorliegen eines Plasmas dienen. Es sind auch andere Größen und deren Kombination und andere Schwellwerte verwendbar.

Die vorgestellte Einrichtung 10 passt während mindestens einer der Phasen für Zündung und Behandlung die verwendete Spannungsform dynamisch an, um einen bestmöglichen Kompromiss zwischen Zündung bzw. Erhaltung des Plasmas und Minimierung oder Maximierung von erwünschten oder unerwünschten Auswirkungen des Zündens und/oder Plasmastroms zu erzielen. Der Plasmasensor überwacht während der Behandlungsphase den Zustand des Plasmas charakterisierende Größen und/oder deren Veränderung, um so eine sich ankündigende Unterbrechung des Plasmas zu erkennen und diese im Idealfall bereits im Vorfeld durch Änderung der Spannungsform zu verhindern.

Die genannten Mechanismen können von der Steuereinrichtung 22 auch während eines Pulspakets P1, P2, P3 genutzt werden. Die Länge jedes Pulspakets wird bei jedem Wechsel der Einstellung gemäß deren Eigenschaften angepasst, um eine gleichbleibende mittlere Leistung sicherzustellen.

Das Verfahren zur Plasmabehandlung ist darauf ausgerichtet, den Hochfrequenzgenerator 15 zum Zünden eines Plasmas an dem Instrument 12 und zum Erhalt desselben jeweils zur Abgabe einer mittelfrequent pulsbreitenmodulierten Hochfrequenzspannung zu veranlassen, deren Tastverhältnis gerade so groß ist, dass es zum Zünden und Erhalt des Funkens ausreicht, nicht aber größer. Dadurch werden Geräusch- und Lichterscheinungen minimiert. Die Festlegung des Tastverhältnisses auf den minimal möglichen Wert kann schrittweise durch ständiges oder gelegentliches Variieren der Einstellung unter Beobachtung der Zündfähigkeit und der Plasmaerhaltung erfolgen. Die durch das Verfahren optimierte Einstellung des Tastverhältnisses kann stufenlos kontinuierlich oder stufenweise durch Auswahl verschiedener Spannungsformen aus einem Vorrat von Spannungsformen erfolgen. Durch Minimierung des Tastverhältnisses der Mittelfrequenz wird die gewünschte Licht- und Geräuscharmut des Behandlungsprozesses hergestellt. Durch gegenläufige Modulation des Tastverhältnisses der Niederfrequenz wird die gewünschte Leistung eingestellt.

### Bezugszeichen:

- 10: Einrichtung
- 11: biologisches Gewebe
- 12: Instruments
- 13: Leitung
- 14: Gerät
- 15: Generator
- 16: Neutralleitung
- 17: Neutralelektrode
- 18: Schlauch
- 19: Kanal
- 20: Elektrode
- 21: Plasma
- 22: Steuereinrichtung
- P1, P2, P3: Pulspakete
- 30 - 46: Blöcke

## Patentansprüche

1. Einrichtung (10) zur Plasmabehandlung
mit einem Generator (15), der zur Erzeugung einer hochfrequenten Wechselspannung (HF) in verschiedenen Einstellungen eingerichtet ist,
mit einem zur Versorgung mit einem Behandlungsstrom an den Generator (15) angeschlossenen oder anschließbaren Instrument (12), das wenigstens eine Elektrode (20) aufweist, die mit der Wechselspannung (HF) gespeist ist, so dass an dieser ein Plasma erzeugbar ist, wobei die bei den verschiedenen Einstellungen erzeugten Wechselspannungen unterschiedliche Zündfähigkeiten und/oder unterschiedliche Plasmaerhaltungsfähigkeiten aufweisen,
mit einer Steuereinrichtung (22), die an den Generator (15) derart angeschlossen ist, dass mittels der Steuereinrichtung (22) die Einstellung des Generators (15) so beeinflussbar ist,
dass der Generator (15) zu Beginn in einer Zündbetriebsart und danach in einer auf den gewünschten Effekt abgestimmten Betriebsart betrieben wird.

2. Einrichtung nach Anspruch 1, **dadurch gekennzeichnet, dass** außerdem
ein Plasmasensor (23) vorgesehen ist, der an den Generator (15) und/oder das Instrument (12) angeschlossen ist, um das Verhalten des Plasmas an dem Instrument (12) festzustellen,
wobei die Steuereinrichtung (22) an den Plasmasensor (23) angeschlossen ist, so dass mittels der Steuereinrichtung (22) die Einstellung des Generators (15) anhand des von dem Plasmasensor (15) erfassten Verhaltens des Plasmas beeinflussbar ist,
wobei die Steuereinrichtung (22) derart ausgebildet ist, dass sie anhand des durch den Plasmasensor (23) erfassten Verhaltens des Plasmas, wenn dieses ein sicheres Zündverhalten und/oder eine sichere Plasmahaltung kennzeichnet, für den Generator (15) eine Einstellung mit verminderter Zündfähigkeit und/oder verminderte Plasmaerhaltungsfähigkeit festlegt, und/oder
dass sie anhand des durch den Plasmasensor (23) erfassten Verhaltens des Plasmas, wenn dieses ein unsicheres Zündverhalten und/oder eine unsichere Plasmahaltung kennzeichnet, für den Generator (15) eine Einstellung mit erhöhter Zündfähigkeit und/oder erhöhter Plasmaerhaltungsfähigkeit festlegt.

3. Einrichtung nach einem der vorstehenden Ansprüche, **dadurch gekennzeichnet, dass** die verschiedenen Einstellungen des Generators diesen veranlassen, Spannungen mit verschiedenen Spannungseigenschaften abzugeben und/oder Spannungen bei verschiedenen Generatoreigenschaften bereitzustellen, wobei die verschiedenen Spannungseigenschaften vorzugsweise die Spannungsgröße und/oder die Modulationsart und/oder den Modulationsgrad und/oder die Spannungsform und/oder das Puls/Pause-Verhältnis und/oder die Frequenz betreffen und/oder dass die Generatoreigenschaften seinen Innenwiderstand betreffen.

4. Einrichtung nach einem der vorstehenden Ansprüche, **dadurch gekennzeichnet, dass** die hochfrequente Wechselspannung (HF) mit einer Mittelfrequenz (MF) gepulst ist, die vorzugsweise kleiner als ein Fünftel der Frequenz der hochfrequenten Wechselspannung (HF), jedoch ebenfalls vorzugsweise größer als ein Zwanzigstel derselben ist, und dass die Steuereinrichtung (22) derart ausgebildet ist, dass sich die Spannungseigenschaften durch verschiedenen Puls/Pause-Verhältnisse der Mittelfrequenz (MF) unterscheiden.

5. Einrichtung nach einem der vorstehenden Ansprüche, **dadurch gekennzeichnet, dass** die hochfrequente Wechselspannung (HF) mit einer Niederfrequenz (NF) gepulst ist, die vorzugsweise mindestens 0,5 Hz und/oder vorzugsweise höchstens 200 Hz und insbesondere vorzugsweise ein bis 100 Hz beträgt.

6. Einrichtung nach einem der vorstehenden Ansprüche, **dadurch gekennzeichnet, dass** die Steuereinrichtung (22) derart ausgebildet ist, dass sie während des Betriebs des Instruments (12) den Generator mit derjenigen unter den verfügbaren Einstellung betreibt, die unter den verschiedenen Einstellung diejenige mit geringstem Nebeneffekt ist und die innerhalb einer Zündversuchszeit zu einer Erzeugung von Plasma führt.

7. Einrichtung nach Anspruch 6, **dadurch gekennzeichnet, dass** die Steuereinrichtung (22) derart ausgebildet ist, dass sie während des Betriebs des Instruments (12) nach Ablauf der Zündversuchszeit den Generator (15) in einer Einstellung mit erhöhtem Nebeneffekt betreibt, wenn mittels des Plasmasensors (23) innerhalb der Zündversuchszeit keine Erzeugung von Plasma erfasst worden ist.

8. Einrichtung nach Anspruch 6, **dadurch gekennzeichnet, dass** die Steuereinrichtung (22) derart ausgebildet ist, dass sie während des Betriebs des Instruments (12) nach zumindest einmaligem, vorzugsweise mehrmaligem erfolgreichen Aufbau des Plasmas innerhalb der Zündversuchszeit den Generator bei dem nächstfolgenden Zündversuch in einer Einstellung mit verringertem Nebeneffekt betreibt.

9. Einrichtung nach Anspruch einem der vorstehenden Ansprüche, **dadurch gekennzeichnet, dass** die Steuereinrichtung (22) derart ausgebildet ist, dass sie während des Betriebs des Instruments (12) den Generator (15) nach Ablauf einer Zündphase in einer Behandlungsphase mit derjenigen Einstellung betreibt, die bei minimalem sonstigem Nebeneffekt den Erhalt des Plasmas gestattet.

10. Einrichtung nach einem der vorstehenden Ansprüche, **dadurch gekennzeichnet, dass** die Steuereinrichtung (22) derart ausgebildet ist, dass sie während des Betriebs des Instruments (12), nach in der Zündphase erfolgtem Aufbau des Plasmas, wenn mittels des Plasmasensors (23) innerhalb Behandlungszeit ein Verlöschen oder eine Instabilität des Plasmas erfasst worden ist, den Generator (15) in der laufenden und/oder in der nachfolgenden Behandlungsphase mit einer Einstellung mit höherer Plasmaerhaltungsfähigkeit und/oder höherem Nebeneffekt betreibt.

11. Einrichtung nach Anspruch 10, **dadurch gekennzeichnet, dass** die Steuereinrichtung (22) derart ausgebildet ist, dass sie die Einstellung des Generators zu einer Einstellung mit niedrigerer Plasmaerhaltungsfähigkeit und/oder niedrigerem Nebeneffekt verändert, wenn während des Betriebs des Instruments (12), nach in der Zündphase erfolgtem Aufbau des Plasmas, der Plasmasensor (23) innerhalb der Behandlungszeit keine Verlöschungstendenz des Plasmas erfasst hat.

12. Einrichtung nach einem der vorstehenden Ansprüche, **dadurch gekennzeichnet, dass** die Steuereinrichtung (22) derart ausgebildet ist, dass sie während des Betriebs des Instruments (12) nach in der Zündphase erfolgtem Aufbau des Plasmas den Generator (15) in einer Behandlungsphase mit einer Spannungsform mit einer Mittelfrequenz (MF) gepulst mit einem Puls/Pause-Verhältnis betreibt, das für die der nächsten Zündphase nachfolgende Behandlungsphase vermindert wird, wenn in wenigstens einer, vorzugsweise mehreren aufeinanderfolgenden Behandlungsphasen mittels des Plasmasensors innerhalb Behandlungszeit ein stabiles Vorhandensein des Plasmas erfasst worden ist, und das vergrößert wird, wenn eine Verlöschungstendenz des Plasmas festgestellt worden ist.

13. Einrichtung nach einem der vorstehenden Ansprüche, **dadurch gekennzeichnet, dass** der Plasmasensor (23) zur Erfassung der Stabilität des Plasmas eine elektrische Kenngröße, insbesondere den zeitlichen Verlauf der elektrischen Kenngröße überwachend ausgebildet ist, wobei die elektrische Kenngröße aus dem zu dem Instrument (12) gelieferten Strom und/oder der zu dem Instrument (12) gelieferten elektrischen Spannung abgeleitet ist.

14. Einrichtung nach einem der vorstehenden Ansprüche, **dadurch gekennzeichnet, dass** die verschiedenen Einstellungen des Generators (15) wenigstens zwei Einstellungen umfassen, die unterschiedliche Zündeigenschaften und/oder unterschiedliche Plasmaerhaltungseigenschaften aufweisen und/oder unterschiedliche Licht- und/oder Schallemissionen verursachen, dabei jedoch im Plasma und/oder im Gewebe eine gleiche mittlere Leistung liefern.

15. Verfahren zur Steuerung einer Einrichtung (10) zur Plasmabehandlung, die einen Hochfrequenzgenerator (15) zur Speisung eines Instruments (12) aufweist, der in verschiedenen Einstellungen betreibbar ist, in denen die von ihm abgegebene Wechselspannung (HF) unterschiedliche Zündeigenschaften und/oder unterschiedliche Plasmaerhaltungseigenschaften und/oder das entstehende Plasma Licht- und Schallemissionen unterschiedlicher Intensität aufweist, wobei der Hochfrequenzgenerator (15) zum Zünden eines Plasmas an dem Instrument (12) und zum Erhalt desselben jeweils eine in einer Einstellung betrieben wird, die unter den verfügbaren Einstellungen diejenige mit geringster Licht- und/oder Schallemission ist, dabei jedoch ein sicheres Zünden und einen sicheren Plasmaerhalt gestattet.
